# EUROPEAN PATENT APPLICATION

(11) **EP 4 458 382 A1**
(43) Date of publication of application: **06.11.2024**
(21) Application number: 22916048.6
(22) Date of filing: 26.12.2022
(51) Int. Cl.: A61L 101/22, A61L 2/20

(54) **STERILIZATION METHOD AND STERILIZATION DEVICE**

(30) Priority: 28.12.2021 JP 2021214560
(71) Applicant: OSAKA UNIVERSITY, Suita-shi Osaka 565-0871 (JP)
(72) Inventor: KITANO Katsuhisa, Suita-shi, Osaka 565-0871 (JP); IKAWA Satoshi, Izumi-shi, Osaka 594-1157 (JP)
(74) Representative: Plasseraud IP
(86) International application number: PCT/JP2022/047999
(87) International publication number: WO 2023/127825

(57) **Abstract**

A sterilization device (1) includes a peroxynitric acid gas generation section (10) and a sterilization treatment section (20). The peroxynitric acid gas generation section (10) generates peroxynitric acid gas obtained by gasifying a peroxynitric acid-containing liquid. In so doing, for example, the peroxynitric acid gas generation section (10) generates a peroxynitric acid mist by ultrasonically atomizing the peroxynitric acid-containing liquid, and causes the peroxynitric acid gas to be generated from the peroxynitric acid mist. The sterilization treatment section (20) applies the peroxynitric acid gas to a sterilization target (22) to sterilize the sterilization target (22).

## Description

### Technical Field

The present invention relates to a sterilization method and a sterilization device.

### Background Art

A sterilization technology such as complete sterilization, disinfection, or sterilization of, for example, medical instruments, living bodies, food containers, and food is one of basic technologies that support modern life. Conventionally, a method for disinfecting, completely sterilizing, or sterilizing, for example, bacteria and viruses can be broadly divided into two types, i.e., a physical method using heat, pressure, or the like, and a chemical method using chemical agents. Note that reducing the number of living microorganisms (living microorganism concentration) is herein referred to as "sterilization".

Examples of the physical method include sterilization with an autoclave using pressurized steam. In the physical method, a target object is limited because a sterilization target is exposed to extreme physical conditions.

The chemical method is used for, for example, precision machines such as an endoscope and heat-sensitive plastic products. Examples of the chemical method include pasteurization using a gas such as ethylene oxide gas (EOG) or a solution of hydrogen peroxide, peracetic acid, hypochlorous acid, or the like. A chemical species used in the chemical method has high toxicity. Thus, the chemical method involves a treatment (e.g., aeration) for rendering the chemical species harmless after sterilization. This consequently requires more cost and time.

Thus, the physical method and the chemical method have both advantages and disadvantages.

In contrast, the inventors of the present invention have developed a sterilization method in which, for example, a peroxynitric acid-containing solution is applied to a sterilization target (see, for example, Patent Literature 1 and Non-patent Literatures 1 to 3). Biocidal activity of a peroxynitric acid-containing liquid disappears in a short time at room temperature. This eliminates the need for detoxification treatment after sterilization.

### Citation List

### [Patent Literature]

[Patent Literature 1]
Japanese Patent No. 6087029

### [Non-patent Literature]

[Non-patent Literature 1]
   Tatsuya Iwaki, Tomoko Ohshima, Tatsuya Tasaki, Yasuko Momoi, Satoshi Ikawa, Katsuhisa Kitano, Takatsugu Yamamoto, Journal of Oral Biosciences "High Microbicidal Effect of Peroxynitric Acid on Biofilm-Infected Dentin in a Root Carious Tooth Model and Verification of Tissue Safety" 2020, Vol. 62, pp. 189-194
[Non-patent Literature 2]
   Takashi Yokoyama, Shinya Miyazaki, Satoshi Ikawa, Yoichi Nakashima, Katsuhisa Kitano, Chemical Research in Toxicology "Kinetics Analysis of the Reactions between Peroxynitric Acid and Amino Acids" (US), 2020, Vol. 33, pp. 1633-1643
[Non-patent Literature 3]
   Takashi Yokoyama, Shinya Miyazaki, Hiroko Akagi, Satoshi Ikawa, Katsuhisa Kitano, Applied and Environmental Microbiology "Kinetics of Bacterial Inactivation by Peroxynitric Acid in the Presence of Organic Contaminants" (US), 2021, Vol. 87, e01860-20

### Summary of Invention

### Technical Problem

Conventionally, a medical instrument, etc. is housed and sterilized in a container while being in a sterilization bag. Further, until immediately before being used, a sterilization target having been sterilized is stored while being in a sterilization bag, and thus can maintain its sterilized state. A sterilization bag is made of nonwoven fabric, glassine paper, or the like, and not only prevents bacteria from passing therethrough, but also prevents substances in the form of droplets from passing therethrough. Thus, a liquid sterilizing agent such as a peroxynitric acid-containing solution has had difficulty in sterilizing a sterilization target that is in a sterilization bag.

The present invention has an object to provide a sterilization method and a sterilization device in each of which peroxynitric acid is used and each of which makes it possible to sterilize even a sterilization target that is in a sterilization bag.

### Solution to Problem

A sterilization method in accordance with an aspect of the present invention is a method in which peroxynitric acid gas is applied to a sterilization target to sterilize the sterilization target.

A sterilization device in accordance with an aspect of the present invention includes a peroxynitric acid gas generation section and a sterilization treatment section. The peroxynitric acid gas generation section generates peroxynitric acid gas obtained by gasifying a peroxynitric acid-containing liquid. The sterilization treatment section applies the peroxynitric acid gas to a sterilization target to sterilize the sterilization target.

### Advantageous Effects of Invention

An aspect of the present invention makes it possible to provide a sterilization method and a sterilization device in each of which peroxynitric acid is used and each of which makes it possible to sterilize even a sterilization target that is in a sterilization bag.

### Brief Description of Drawings

Fig. 1 is a view illustrating, as an example of a sterilization device in accordance with an embodiment, a sterilization device that generates peroxynitric acid gas by a nebulizer method.
Fig. 2 is a graph showing a result of a first sterilization test using a large container.
Fig. 3 is a graph showing a result of a second sterilization test using a large container.
Fig. 4 is a table showing a result of a third sterilization test using a large container.
Fig. 5 is a table showing a result of a fourth sterilization test using a large container.
Fig. 6 is a graph showing a result of a fifth sterilization test using a small container.
Fig. 7 is a table showing microbicidal activity determined from a survival curve shown in Fig. 6.
Fig. 8 is a graph showing a result of a sixth sterilization test using a contaminated pig skin model.
Fig. 9 is a view illustrating a peroxynitric acid gas generation section that generates peroxynitric acid gas by gas mixture spraying.
Fig. 10 is a graph showing half-time of a peroxynitric acid solution.

### Description of Embodiments

### <Description of outline of embodiments>

The present invention can have various embodiments as below.

That is, a sterilization method in accordance with an embodiment is a method in which peroxynitric acid gas is applied to a sterilization target to sterilize the sterilization target. As described earlier, reducing the number of living microorganisms (living microorganism concentration) is herein referred to as "sterilization".

The peroxynitric acid gas is, for example, vaporized gas generated from a peroxynitric acid mist obtained by atomizing a peroxynitric acid (PNA, HOONO₂)-containing liquid. Note that the peroxynitric acid-containing liquid may be generated by, for example, a method disclosed in the above-listed Patent Literature 1. For example, the peroxynitric acid-containing liquid is generated by mixing a peroxide (hydrogen peroxide, etc.) and a nitrite under a strongly acidic condition where pH is 2 or lower.

Examples of a method for generating the peroxynitric acid mist include a method in which the peroxynitric acid-containing liquid is atomized by ultrasonic atomization. Alternatively, for example, the peroxynitric acid mist may be generated by atomizing the peroxynitric acid-containing liquid by spraying the peroxynitric acid-containing liquid. Further, for example, a peroxynitric acid gas-containing gas may be generated by bubbling the peroxynitric acid-containing liquid with gas (air). Alternatively, for example, the peroxynitric acid gas-containing gas may be generated by gasifying the peroxynitric acid-containing liquid by evaporating the peroxynitric acid-containing liquid.

Herein, a mist is a fine droplet and refers to one that cannot pass through a gas-permeable material, such as glassine paper used in a sterilization bag. The gas-permeable material prevents a peroxynitric acid mist from passing therethrough. However, causing the peroxynitric acid mist to have a more minute size results in a larger surface area with respect to the same volume. This makes it possible to efficiently generate, in a gas containing the peroxynitric acid mist, peroxynitric acid gas that can pass through the gas-permeable material. That is, the peroxynitric acid gas passes through the sterilization bag from the gas containing the mist and present around the sterilization bag. Thus, gasification refers to making a peroxynitric acid molecule contained in a liquid in the form of a mist or the like into a state in which the peroxynitric acid molecule can pass through the gas-permeable material described earlier.

The following description will discuss details of an example of a peroxynitric acid gas generation method.

### <Ultrasonic atomization>

Examples of a specific method of ultrasonic atomization include a nebulizer method, an atomizer method, and a mesh method. In the nebulizer method, a second container containing a peroxynitric acid-containing liquid (hereafter referred to as a peroxynitric acid solution) is provided in a first container containing a liquid such as water. An ultrasonic transducer provided in the first container causes the liquid such as water in the first container to vibrate, so that the peroxynitric acid solution in the second container is atomized. In the atomizer method, an ultrasonic transducer is used to directly cause a peroxynitric acid solution to vibrate, so that the peroxynitric acid solution is atomized. In the mesh method, a peroxynitric acid solution is atomized by supplying the peroxynitric acid solution to a metal mesh while causing the metal mesh to vibrate.

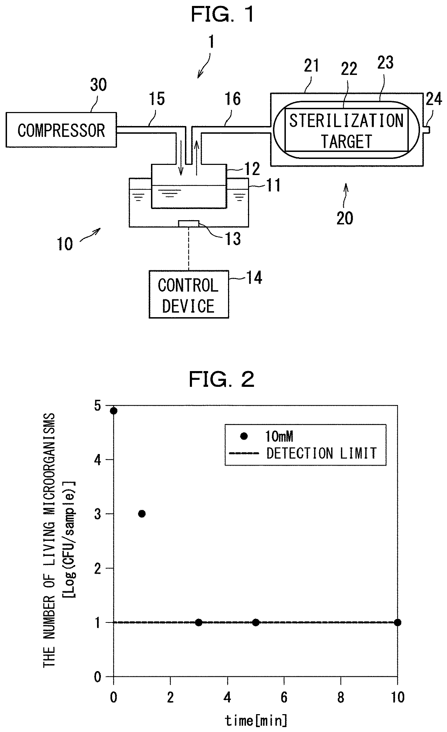

Fig. 1 is a view illustrating, as an example of a sterilization device in accordance with an embodiment, a sterilization device 1 that generates peroxynitric acid gas by a nebulizer method. The sterilization device 1 includes a peroxynitric acid gas generation section 10 and a sterilization treatment section 20.

The peroxynitric acid gas generation section 10 includes a first container 11, a second container 12, an ultrasonic transducer 13, and a control device 14. The ultrasonic transducer 13 is provided in a bottom part of the first container 11. A liquid such as water is contained in the first container 11. Further, the second container 12 is provided in the first container 11. A peroxynitric acid solution is contained in the second container 12. To the second container 12 in a closed state, a first pipe 15 and a second pipe 16 are connected.

The control device 14 controls operation of the ultrasonic transducer 13. The control device 14 includes a processor such as a central processing unit (CPU) or a micro processing unit (MPU), and a memory such as a read only memory (ROM) and a random access memory (RAM). The processor controls the operation of the ultrasonic transducer 13 by executing a computer program stored in the memory.

The sterilization treatment section 20 includes a sterilization container 21. In the illustrated example, the sterilization container 21 is a box-like container, and may have, for example, any shape and any capacity. Further, a pipe such as the second pipe 16 may be used as the sterilization container 21. The sterilization container 21 houses therein a sterilization target 22. The sterilization target 22 is any of a medical instrument, a food container, and the like. Note that the sterilization target 22 may be housed in the sterilization container 21 while being in a sterilization bag 23. In the illustrated example, the sterilization target 22 is assumed to be in the sterilization bag 23. The sterilization bag 23 is made of a gas-permeable material that prevents bacteria and a liquid from passing therethrough and that allows a gas to pass therethrough, such as nonwoven fabric or glassine paper. The sterilization container 21 communicates with the second container 12 via the second pipe 16. Further, the sterilization container 21 has an air outlet 24 that is open.

A compressor 30 feeds air to the second container 12 via the first pipe 15. Note that a means for feeding air to the second container 12 is not limited to the compressor 30, and may be a pump, a fan, or the like.

Next, the following description will discuss operation of the sterilization device 1. In accordance with a control command from the control device 14, the ultrasonic transducer 13 vibrates, so that the liquid such as water in the first container 11 vibrates. This results in atomization of the peroxynitric acid solution in the second container 12, so that a peroxynitric acid mist is generated. The peroxynitric acid mist is a fine droplet of the peroxynitric acid solution, and vaporized gas (i.e., peroxynitric acid gas) is generated by evaporation or deaeration of the peroxynitric acid mist. That is, a gas containing the peroxynitric acid mist contains the peroxynitric acid gas. Together with gas (e.g., air) fed from the compressor 30, a gas containing the peroxynitric acid mist and the peroxynitric acid gas flows through the second pipe 16 and is introduced into the sterilization container 21. The peroxynitric acid gas introduced into the sterilization container 21 is capable of passing through the sterilization bag 23, and sterilizes the sterilization target 22 in the sterilization bag 23. The gas containing the peroxynitric acid mist and the peroxynitric acid gas and introduced into the sterilization container 21 is finally discharged outside through the air outlet 24.

The sterilization device 1 in the illustrated example is configured to use the compressor 30 to feed air to the second container 12. Note, however, that the sterilization device 1 may alternatively be configured not to feed air, i.e., not to include the compressor 30 and the first pipe 15. Further, the sterilization device 1 in the illustrated example is configured to discharge the peroxynitric acid gas in the sterilization container 21 outside through the air outlet 24. Note, however, that the sterilization device 1 may alternatively be configured to recirculate the peroxynitric acid gas into the second container 12.

The following description will discuss, with reference to Figs. 2 to 5, results of sterilization tests each using the sterilization device 1 that generates peroxynitric acid gas by a nebulizer method. Here, the sterilization tests were carried out with use of the sterilization device 1 that includes (i) the peroxynitric acid gas generation section 10, which is as illustrated in Fig. 1 and which generates the peroxynitric acid gas by a nebulizer method and (ii) the sterilization treatment section 20 in which the sterilization container 21 having a capacity of 90 L is used. The peroxynitric acid solution was used in an amount of approximately 15 mL per minute in the peroxynitric acid gas generation section 10. The peroxynitric acid mist and the peroxynitric acid gas in the sterilization container 21 were configured to be recirculated into the second container 12 instead of being discharged outside through the air outlet 24.

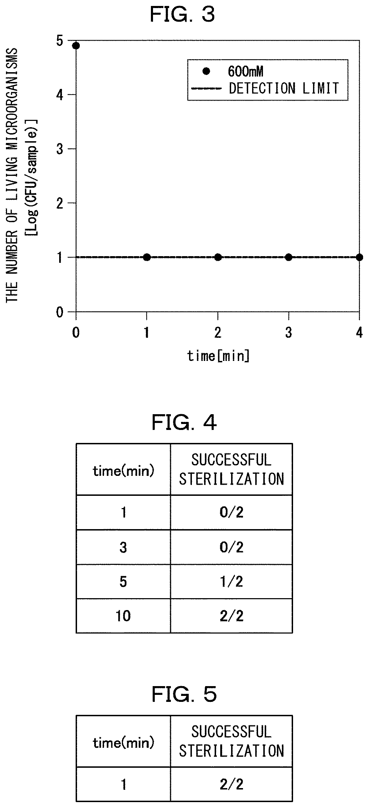

Fig. 2 is a graph showing a result of a first sterilization test using a large container. Fig. 3 is a graph showing a result of a second sterilization test using a large container. As the sterilization target 22, a biological indicator (BI) for determining a sterilization effect was housed in the sterilization container 21 having a capacity of 90 L. The BI is one in which 10⁵ CFU bacterial spores (Geobacillus stearothermophilu) are supported on a stainless steel disk and the stainless steel disk is in the sterilization bag 23 made of glassine paper. Molar concentrations of the peroxynitric acid solution to be placed in the second container 12 were set to 10 mM (Fig. 2) and 600 mM (Fig. 3). After the sterilization test, the BI was removed from the sterilization container 21 and cultured, and the number of living microorganisms was examined after the culture (a CFU assay). In a case where the number of living microorganisms is equal to or lower than a detection limit, it is considered that the BI has been successfully sterilized. As illustrated in Fig. 2, in a case where the peroxynitric acid solution had a molar concentration of 10 mM, the BI was sterilized in 3 minutes. As illustrated in Fig. 3, in a case where the peroxynitric acid solution had a molar concentration of 600 mM, the BI was sterilized in 1 minute.

Fig. 4 is a table showing a result of a third sterilization test using a large container. Fig. 5 is a table showing a result of a fourth sterilization test using a large container. The BI is one in which 10⁶ CFU bacterial spores (Geobacillus stearothermophilu) are supported on a stainless steel disk and the stainless steel disk is in the sterilization bag 23 made of glassine paper. Molar concentrations of the peroxynitric acid solution to be placed in the second container 12 were set to 10 mM (Fig. 4) and 600 mM (Fig. 5). After sterilization, the BI was removed from the sterilization container 21 and cultured, and a culture solution was examined for turbidity (herein referred to as an on-off test). The culture solution in which even one bacterium remains is turbid. This makes it possible to say that the on-off test has a lower detection limit than the CFU assay and that it is difficult to sterilize the BI. Nevertheless, as illustrated in Fig. 4, in a case where the peroxynitric acid solution had a molar concentration of 10 mM, the BI was sterilized in 10 minutes. As illustrated in Fig. 5, in a case where the peroxynitric acid solution had a molar concentration of 600 mM, the BI was sterilized in 1 minute.

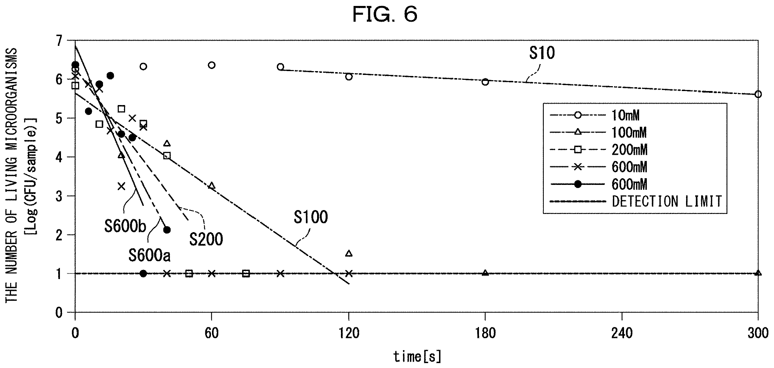

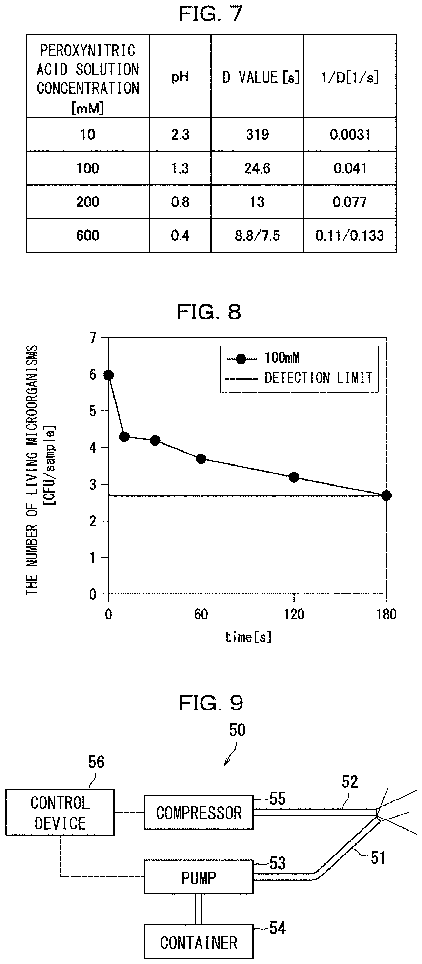

Subsequently, the following description will discuss, with reference to Figs. 6 and 7, a result of a sterilization test using the sterilization device 1 that generates peroxynitric acid gas by a nebulizer method. The capacity of the sterilization container 21 was as large as 90 L in the sterilization tests in Figs. 2 to 5, whereas the capacity of the sterilization container 21 was as small as approximately 0.3 L in the sterilization test in Figs. 6 and 7. The peroxide solution was used in an amount of approximately 3 mL per minute in the peroxynitric acid gas generation section 10. The peroxynitric acid gas in the sterilization container 21 is discharged outside through the air outlet 24 instead of being recirculated into the second container 12.

Fig. 6 is a graph showing a result of a fifth sterilization test using a small container. The BI is one in which 10⁵ CFU bacterial spores (Geobacillus stearothermophilu) are supported on a stainless steel disk and the stainless steel disk is in the sterilization bag 23 made of glassine paper. Types of the molar concentration of the peroxynitric acid solution to be placed in the second container 12 were set to the following four types: 10 mM, 100 mM, 200 mM, and 600 mM. Note that only the sterilization test at 600 mM was carried out twice. The CFU assay resulted in obtainment of (i) a survival curve S10 in the case of the peroxynitric acid solution having a molar concentration of 10 mM, (ii) a survival curve S100 in the case of the peroxynitric acid solution having a molar concentration of 100 mM, (iii) a survival curve S200 in the case of the peroxynitric acid solution having a molar concentration of 200 mM, and (iv) survival curves S600a and S600b in the case of the peroxynitric acid solution having a molar concentration of 600 mM. These survival curves have shown that the peroxynitric acid solution having a higher molar concentration achieves higher microbicidal activity.

Fig. 7 is a table showing microbicidal activity determined from each of the survival curves S10, S100, S200, S600a, and S600b that are shown in Fig. 6. A peroxynitric acid solution concentration [mM] shown in the table is the molar concentration of the peroxynitric acid solution placed in the second container 12. Note that a lower molar concentration results in higher pH because the molar concentration was reduced by diluting an undiluted solution of the peroxynitric acid solution. A D value [s] is a time which is calculated from a survival curve and at which the number of living microorganisms reaches 1/10. 1/D [1/s] is a reciprocal of the D value and indicates microbicidal activity. The table has shown that microbicidal activity is proportional to the molar concentration of the peroxynitric acid solution and that the peroxynitric acid solution having a higher molar concentration achieves higher microbicidal activity.

The sterilization test results shown in Figs. 2 to 7 have shown that the BI which was in the sterilization bag 23 was sterilized on an order of several seconds to several minutes. It is concluded that the BI is sterilized by the peroxynitric acid gas because the sterilization bag 23 prevents the peroxynitric acid mist from passing therethrough and allows the peroxynitric acid gas to pass therethrough. That is, the peroxynitric acid gas is a sterilization factor.

As described later, peroxynitric acid in a gaseous state has a shorter half-time than peroxynitric acid in a liquid state. Thus, the peroxynitric acid in the gaseous state has a less sustained sterilizing effect than the peroxynitric acid in the liquid state. However, in a state in which air and the peroxynitric acid mist are mixed, the peroxynitric acid gas continues to be supplied to the air by evaporation or volatilization of the peroxynitric acid mist. This makes it possible to maintain the sterilization effect brought about by peroxynitric acid gas. That is, as compared with a system that supplies only the peroxynitric acid gas, a system that supplies the peroxynitric acid gas together with the peroxynitric acid mist allows a high sterilization effect brought about by the peroxynitric acid gas to be maintained for a longer time, and enables sterilization in a larger space. Actually, the sterilization test results shown in Figs. 2 to 5 have shown that, even in a case where a large container was used as the sterilization container 21, the BI was successfully sterilized on an order of several minutes, and sufficient microbicidal activity was exhibited.

The following description will discuss characteristics of each of the peroxynitric acid gas and the peroxynitric acid mist in detail.

In a case where peroxynitric acid in a gasified state is supplied to a sterilization target, a concentration of the peroxynitric acid continues to decrease from a time point at which the peroxynitric acid is gasified. Thus, in a case where it takes a longer time until the peroxynitric acid is applied to the sterilization target, the concentration of the peroxynitric acid gas may decrease to not higher than a concentration that is effective for sterilization. In contrast, in a case where a gas containing the peroxynitric acid mist is supplied to the sterilization target, even if the peroxynitric acid gas, which has a short half-time, is deactivated, the peroxynitric acid gas is continuously supplied due to gas-liquid equilibrium from the peroxynitric acid mist, which has a relatively long half-time. Thus, the peroxynitric acid gas the concentration of which is close to a saturation concentration is continuously maintained at or near the peroxynitric acid mist. This prevents or reduces a decrease in concentration of the peroxynitric acid gas, makes it possible to maintain a long-term sterilization action caused by the peroxynitric acid gas having a high concentration, and allows sterilization to be carried out with respect to a large-volume space, such as a cell culture isolator.

In comparison between (a) a case where the gas containing the peroxynitric acid mist is applied to the sterilization target and (b) a case where the peroxynitric acid solution is sprayed, as it is, on the sterilization target by spraying, the case where the gas containing the peroxynitric acid mist is applied to the sterilization target has an advantage that, even if the peroxynitric acid mist does not come into contact with the sterilization target, the peroxynitric acid gas generated around the sterilization target enables sterilization. That is, since it is unnecessary for the entire surface of the sterilization target to be sterilized to be completely covered with the peroxynitric acid solution, a relatively small amount of the peroxynitric acid solution makes it possible to achieve sterilization. This enables a significant reduction in amount of a chemical solution used.

In a case where the sterilization target has a fine structure, it is difficult to apply, to the sterilization target, the peroxynitric acid solution as a droplet. In contrast, the peroxynitric acid gas can be expected to have a reliable sterilization effect because diffusion in a gas phase allows a peroxynitric acid molecule to reach the fine structure.

Subsequently, the following description will discuss, with reference to Fig. 8, a result of a sterilization test using the sterilization device 1 that generates peroxynitric acid gas by a nebulizer method. In the sterilization test shown in Fig. 8, with the aim of application to biological disinfection, a contaminated pig skin model was sterilized by the nebulizer method.

Fig. 8 is a graph showing a result of a sixth sterilization test using a contaminated pig skin model. A 10⁶ CFU spore solution (Bacillus subtilis) was applied to pig skin that had been shaved, cleaned, and subjected to sterilization treatment in advance, and the pig skin was dried so as to be a contaminated model. Such a contaminated pig skin model was regarded as the sterilization target 22 and housed in the sterilization container 21 having a capacity of approximately 0.3 L. In so doing, a surface of the contaminated pig skin model was made perpendicular to a direction in which a gas containing the peroxynitric acid gas and the peroxynitric acid mist flowed. A molar concentration of the peroxynitric acid solution to be placed in the second container 12 was set to 100 mM. After the sterilization test, a stomachere was used to collect bacteria on not only a front surface but also a back surface of the contaminated pig skin model, and the CFU assay was carried out. As shown in Fig. 8, a longer exposure time resulted in a reduction in number of living microorganisms, and the contaminated pig skin model was sterilized in 3 minutes.

The sterilization test result shown in Fig. 8 has shown that the peroxynitric acid gas was effective in disinfection of living organisms. There are various applications such as disinfection of fingers (e.g., application to a handwashing device), disinfection of wounds (e.g., a trauma, a bedsore, a burn, and an ulcer), and disinfection of a part to be incised before surgery. Note that safety of the concentration of the 100 mM peroxynitric acid solution has been verified in an animal experiment. A method in which the peroxynitric acid gas is used as in the present sterilization test is considered to be safer than a method in which the peroxynitric acid solution is applied directly to the sterilization target 22. Thus, the peroxynitric acid gas can be expected to have a sterilization effect for the purpose of preventing and treating infectious diseases.

In the first to sixth sterilization tests above, the diluted peroxynitric acid solution was used. For example, peroxynitric acid is chemically synthesized at approximately pH 0 to generate an undiluted solution at 1 M, and the undiluted solution is diluted 10 times to generate a peroxynitric acid solution at 100 mM and pH 1. Alternatively, the undiluted solution is diluted 100 times to generate a peroxynitric acid solution at 10 mM and pH 2.

Given that a molar concentration which is close to the molar concentration 1 M of the undiluted solution results in lower pH, the peroxynitric acid solution is difficult to atomize. Further, from the peroxynitric acid solution having a high concentration, air bubbles formed by oxygen gas generated by decomposition of the peroxynitric acid are generated on a container surface. This interferes with ultrasonic atomization. Thus, as pretreatment for sterilization, the control device 14 generates ultrasonic waves at kilohertz frequencies (e.g., 50 kHz) by controlling the operation of the ultrasonic transducer 13. This removes air bubbles contained in the peroxynitric acid solution (i.e., deaeration). During sterilization, the control device 14 generates ultrasonic waves at megahertz frequencies suitable for atomization by controlling the operation of the ultrasonic transducer 13. The peroxynitric acid solution that has been deaerated before sterilization is easier to atomize. In order to remove air bubbles generated during atomization, atomization and removal of air bubbles may be carried out alternately. Note that, in a case where the peroxynitric acid solution has a low molar concentration and is easy to atomize, sterilization may be carried out by generating ultrasonic waves at megahertz frequencies without carrying out deaeration.

In a case where the peroxynitric acid solution is difficult to atomize, the peroxynitric acid solution may be vacuum-deaerated as pretreatment for sterilization. The peroxynitric acid solution that has been vacuum-deaerated before sterilization is easier to atomize.

### <Spraying>

Examples of a specific method of spraying include one-component spraying and gas mixture spraying. In the one-component spraying, a peroxynitric acid mist is generated by spraying a peroxynitric acid solution, and peroxynitric acid gas is generated from the peroxynitric acid mist. In the gas mixture spraying, a peroxynitric acid mist is generated by mixing a peroxynitric acid solution with air, and peroxynitric acid gas is generated from the peroxynitric acid mist.

Fig. 9 is a view illustrating a peroxynitric acid gas generation section 50 that generates peroxynitric acid gas by gas mixture spraying. The peroxynitric acid gas generation section 50 includes a first nozzle 51, a second nozzle 52, a pump 53, a container 54, a compressor 55, and a control device 56.

A peroxynitric acid solution is contained in the container 54. The pump 53 sends the peroxynitric acid solution in the container 54 to the first nozzle 51. The pump 53 may apply pressure to the peroxynitric acid solution during sending of the peroxynitric acid solution to the first nozzle 51. The first nozzle 51 sprays the peroxynitric acid solution from its tip.

The compressor 55 feeds air to the second nozzle 52. Note that a means for feeding air to the second nozzle 52 is not limited to the compressor 55, and may be a pump or the like. The second nozzle 52 blows out air from its tip. The tip of the first nozzle 51 and the tip of the second nozzle 52 are disposed in close proximity to each other. This brings the peroxynitric acid solution emitted from the first nozzle 51 into collision with the air emitted from the second nozzle 52, so that a peroxynitric acid mist is generated. Evaporation of the peroxynitric acid mist results in formation of vaporized gas (i.e., peroxynitric acid gas). That is, a gas containing the peroxynitric acid mist contains the peroxynitric acid gas. The gas containing the peroxynitric acid mist and the peroxynitric acid gas is introduced into the sterilization treatment section 20 (see Fig. 1). The sterilization target 22 that is housed in the sterilization container 21 may be in the sterilization bag 23.

Gas blown out from the second nozzle 52 may be heated by a heating heater or the like. A temperature of the gas containing the peroxynitric acid mist is increased in a case where hot air blown out from the second nozzle 52 is mixed with the peroxynitric acid mist sprayed from the first nozzle 51. An increase in temperature of the gas increases saturated vapor pressure. This results in an increase in concentration of the peroxynitric acid gas. Further, the increase in temperature brings about an effect of increasing a sterilization rate (i.e., chemical reaction rate). This makes it possible to efficiently sterilize the sterilization target 22. A method of spraying the peroxynitric acid solution is suitable particularly for sterilization of a food container such as a polyethylene terephthalate (PET) bottle. Efficient sterilization by heating makes it possible to expect sterilization to be carried out in a short time, and also leads to a reduction in cost required for sterilization.

The control device 56 may be configured such that spraying of the peroxynitric acid solution from the first nozzle 51 is alternated with blowing out of heated air from the second nozzle 52 by controlling operation of the pump 53 and the compressor 55. The control device 56 causes hot air to be blown out first so as to increase an ambient temperature in the sterilization container 21, and then causes the peroxynitric acid solution to be sprayed so as to sterilize the sterilization target 22 with the peroxynitric acid gas. Subsequently, by causing hot air to be blown out so as to create a high-temperature environment, the control device 56 dries the sterilization target 22 and renders the peroxynitric acid harmless.

Note that the control device 56 may cause hot air to be blown out at all times, and may cause the peroxynitric acid solution to be sprayed for a short time in the intervals between the times.

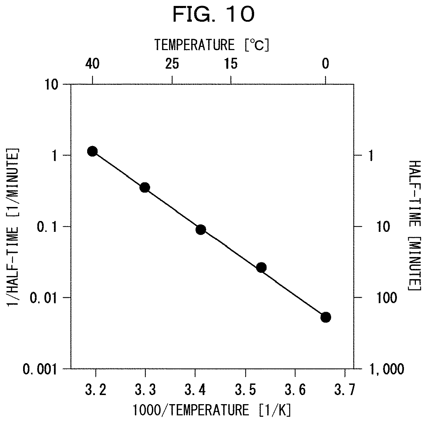

As described in detail in Fig. 10, a lifetime of the synthesized peroxynitric acid has temperature dependency, and a high temperature causes an increase in rate of decomposition of the peroxynitric acid, so that the peroxynitric acid is changed into nitric acid in a shorter time (i.e., deactivated). Thus, in a case where hot air is passed through the second nozzle 52, an increase in temperature of the peroxynitric acid solution that passes through the first nozzle 51 is preferably prevented by carrying out thermal insulation by, for example, keeping a sufficient distance between the second nozzle 52 and the first nozzle 51 as illustrated in Fig. 9. Note that the first nozzle 51 and the second nozzle 52 may be integrally held in a case where normal or low temperature air is passed through the second nozzle 52.

### <Removal of peroxynitric acid mist, etc.>

The gas generated in the peroxynitric acid gas generation section 10, 50 contains not only the peroxynitric acid gas but also the peroxynitric acid mist, etc. Further, in a case where nitrous acid and a peroxide (e.g., hydrogen peroxide) are mixed to generate the peroxynitric acid solution, the gas generated in the peroxynitric acid gas generation section 10, 50 can contain unnecessary chemical substances such as hydrogen peroxide, nitrite, and nitrate. Even if the gas generated in the peroxynitric acid gas generation section 10, 50 contains these unnecessary chemical substances, the sterilization effect of the peroxynitric acid gas is unchanged. Note, however, that there is a problem of white precipitation as salt on a surface of, for example, the sterilization target 22 which has been sterilized. In that case, for example, cleaning treatment after sterilization is required.

Thus, a separation device may be provided between the peroxynitric acid gas generation section 10, 50 and the sterilization treatment section 20 (e.g., on the second pipe 16) in order to remove the peroxynitric acid mist and the unnecessary chemical substances from the gas generated by the peroxynitric acid gas generation section 10, 50, and to apply only the peroxynitric acid gas to the sterilization target 22.

The separation device desirably removes a component(s) other than the peroxynitric acid gas from the gas generated in the peroxynitric acid gas generation section 10, 50. For example, the separation device is made of a nonwoven fabric filter that adsorbs mist. Further, for example, the separation device may be a cyclone that subjects mist and gas to centrifugation.

The sterilization bag 23 may be double-layered and subjected to sterilization treatment in order to prevent adhesion of salt to a surface of the sterilization bag 23 while sterilizing an interior of the sterilization bag 23. Nonwoven fabric, glassine paper, or the like of which the sterilization bag 23 is made prevents a liquid from passing therethrough and allows only gas from passing therethrough. Thus, in a case where the sterilization bag 23 is double-layered, even if salt is deposited on an outer-side sterilization bag 23, only the peroxynitric acid gas penetrates into the sterilization bag 23. This prevents the interior of the sterilization bag 23 from being contaminated with salt. Further, since the mist and the gas can be separated in close proximity to the sterilization target 22, the concentration of the peroxynitric acid gas can be kept high.

Note that, in a case where a single-layer sterilization bag 23 is used, it is only necessary to wash the sterilization bag 23 with water after the sterilization treatment to remove salt deposited on the surface of the sterilization bag 23. In a case where a double-layer sterilization bag 23 is used, it is only necessary to remove the outer-side sterilization bag 23 after the sterilization treatment, and it is unnecessary to wash the sterilization bag 23 with water. Even if the outer-side sterilization bag 23 is removed, sterilization of the sterilization target 22 is maintained by an inner-side sterilization bag 23.

Note that, in order to prevent salt deposition, an ion exchange resin, etc. may be used to carry out desalting treatment with respect to the peroxynitric acid solution. Use of the peroxynitric acid solution that has been subjected to the desalting treatment makes it possible to generate a peroxynitric acid mist containing no salt component.

The peroxynitric acid solution is desirably applied to the sterilization target under an acidic condition. Thus, in some cases, pH is adjusted by addition of acid (e.g., nitric acid) during synthesis of the peroxynitric acid solution. In that case, the acid is also mixed with a resulting peroxynitric acid mist, and the acid can adhere to a surface of the sterilization target 22. For example, a hard-to-volatilize acid may be used to synthesize the peroxynitric acid solution in order to save time and effort required for cleaning the acid that has adhered to the surface of the sterilization target 22. The hard-to-volatilize acid is an acid having a low vapor pressure, and is exemplified by sulfuric acid. Sulfuric acid has a vapor pressure (0.0067 Pa at 25°C) that is lower than a vapor pressure (6.4 kPa at 20°C) of nitric acid. Adjustment of pH by addition of sulfuric acid during synthesis of the peroxynitric acid solution may make it difficult for the acid as an impurity to be dispersed during gasification. Examples of the hard-to-volatilize acid include not only sulfuric acid but also phosphoric acid (4 Pa at 20°C) and periodic acid (which is a solid and thus does not volatilze).

Further, in some cases, a liquid in which sodium nitrite in powder form is dissolved instead of nitrous acid is used to synthesize a peroxynitric acid solution by chemically reacting nitrous acid and hydrogen peroxide. This is because nitrous acid is unstable by itself. However, sodium nitrite, which contains Na, leads to salt deposition. Thus, in order to prevent salt deposition, it is possible to, for example, use nitrous acid gas generated from an acidified sodium nitrite solution, or use nitrous acid gas stored in a cylinder. Furthermore, nitrous acid gas and hydrogen peroxide gas may be mixed to generate peroxynitric acid gas.

### <Half-time of peroxynitric acid gas>

Fig. 10 is a graph showing half-time of a peroxynitric acid solution. As shown in the graph, the peroxynitric acid solution that has a lower temperature (which may be ambient temperature) has a longer half-time. For example, the peroxynitric acid solution that has pH of 2.9 has a half-time of 132 minutes at 2°C of the temperature of the peroxynitric acid solution, has a half-time of 26 minutes at 10°C of the temperature of the peroxynitric acid solution, has a half-time of 7.8 minutes at 20°C of the temperature of the peroxynitric acid solution, has a half-time of 2 minutes at 30°C of the temperature of the peroxynitric acid solution, and has a half-time of 0.6 minutes at 40°C of the temperature of the peroxynitric acid solution. The half-time of the peroxynitric acid solution thus depends on temperature.

Regarding a half-time of peroxynitric acid gas, the following literature discloses a formula. For example, the peroxynitric acid gas has a half-time of 97 seconds under conditions of 1 atmospheric pressure and 4.55°C (277.7 K). Further, the half-time is 12 seconds under conditions of 1 atmospheric pressure and 24.85°C (298 K).
Richard A. Graham, Arthur M. Winer, and James N. Pitts Jr., The Journal of Chemical Physics "Pressure and temperature dependence of the unimolecular decomposition of HO2NO2" (US), 1978, Vol. 68, p. 4505

Thus, the peroxynitric acid mist and the peroxynitric acid gas are decomposed at a higher rate at a higher temperature, and are changed into nitric acid in a shorter time (i.e., deactivated). Thus, without detoxification treatment carried out after sterilization, the peroxynitric acid that has adhered to the surface of the sterilization target 22 is deactivated merely by storing the sterilization target 22 at room temperature. In particular, since the peroxynitric acid gas has a shorter half-time than the peroxynitric acid mist, it is less necessary to carry out detoxification treatment after sterilization using only the peroxynitric acid gas.

In order to prevent the peroxynitric acid solution in the second container 12 from being thermally deactivated, the sterilization device 1 may have a structure that keeps the temperature of the peroxynitric acid solution low. For example, the peroxynitric acid solution in the second container 12 is cooled by adding a heat exchanger to the second container 12 and circulating an external cooling solution in the heat exchanger. Further, for example, the peroxynitric acid solution is cooled by connecting, to the second container 12, an external tank provided with a heat exchanger, and circulating the peroxynitric acid solution between the second container 12 and the external tank. The structure that keeps the temperature of the peroxynitric acid solution low may be a structure other than the above example.

Note that the peroxynitric acid in a liquid state has a longer half-time than the peroxynitric acid in a gas state under an identical temperature condition. Thus, by transporting and supplying, to the sterilization target 22, the peroxynitric acid gas as a gas containing the peroxynitric acid mist, a decrease in concentration of the peroxynitric acid gas is prevented or reduced, and a sterilization effect can be expected to be brought about in a wider range by the peroxynitric acid gas having a high concentration, as compared with supply of only the peroxynitric acid gas. This enables application to sterilization of large spaces such as an operating room, a food manufacturing plant, and a plant factory. Further, a fumigant may be used to sterilize seeds and agricultural products. Note, however, that the peroxynitric acid gas or the peroxynitric acid mist may be usable instead of the fumigant.

### <Detoxification treatment>

As described above, the peroxynitric acid gas and the peroxynitric acid mist each have a short half-time at room temperature. This makes it less necessary to carry out detoxification treatment after sterilization. In a case where the detoxification treatment is carried out after sterilization, aeration, ultraviolet irradiation, or the like is carried out. For example, the following literature indicates that peroxynitric acid is decomposed by ultraviolet irradiation.
Helene MacLeod, Gregory P. Smith, David M. Golden, Journal of Geophysical Research Atmospheres "Photodissociation of pernitric acid (HO2NO2) at 248 nm" (US), 1988, Vol. 93, pp. 3813-3823

### <pH of peroxynitric acid solution>

As disclosed in the above Patent Literature 1, in order to produce peroxynitric acid by a reaction of peroxide (hydrogen peroxide, etc.) and nitrite, it is a prerequisite that the pH value is 2 or lower of strong acid. Lower pH allows the peroxynitric acid to be synthesized with higher efficiency. Further, the peroxynitric acid that has lower pH is more stable, and an increase in pH causes the peroxynitric acid to be decomposed quickly, so that the peroxynitric acid has a shorter half-time.

Nitric acid has pKa of approximately -1.4, and peroxynitric acid has pKa of approximately 5.85. At pH higher than pKa, the peroxynitric acid solution is generally difficult to vaporize due to a high proportion of its presence as an ionic body. That is, by causing the peroxynitric acid solution to have pH of 5.85 or lower, it is possible to make it difficult to vaporize the nitric acid while preferentially vaporizing the peroxynitric acid to obtain high microbicidal activity. Thus, the pH of the peroxynitric acid solution used to generate the peroxynitric acid gas is preferably set to -1.4 or higher and 5.85 or lower.

Further, as disclosed in the above Patent Literature 1, it is considered that the peroxynitric acid (HOONO₂) synthesized by the chemical reaction generates, for example, protons (H⁺), superoxide anion radicals (O₂⁻·), and nitrogen dioxide (NO₂·), and these are diffused in the solution. As shown in Formula (1) below, the superoxide anion radicals react with the protons in the solution to generate hydroperoxy radicals (HOO·).

O₂⁻· + H⁺ ←→ HOO· (1)

The hydroperoxy radicals shown in Formula (1) above have an extremely short lifetime, but have more powerful microbicidal activity than the superoxide anion radicals. Formula (1) expresses an equilibrium reaction, and an equilibrium relationship depending on pH of the solution is established. In a case where the protons have a high concentration, the hydroperoxy radicals have a high concentration. That is, a dissociation constant (acid dissociation constant) representing an equilibrium constant of this equilibrium reaction formula pKa is 4.8.

In a case where pH is higher than 4.8, the concentration of the superoxide anion radicals is increased, and the hydroperoxy radicals have a low concentration. In a case where pH is 4.8 or lower, the concentration of the hydroperoxy radicals is increased, and the hydroperoxy radicals exert extremely powerful microbicidal activity. High microbicidal activity can be obtained also for the peroxynitric acid gas by increasing microbicidal activity by setting the pH of the peroxynitric acid solution to 4.8 or lower.

The peroxynitric acid solution that has lower pH has higher microbicidal activity. Note, however, that even a reduction in pH to approximately 3 or lower less changes the sterilization effect. Further, pH of 0.5 or lower may cause a problem of metal corrosion. It is therefore practical to set, to approximately 2 to approximately 5.85, the pH of the peroxynitric acid solution used to generate the peroxynitric acid gas.

As described above, the sterilizing effect of the peroxynitric acid increases sharply under an acidic condition (pH of 4.8 or lower). Thus, by actively supplying acid gas to the sterilization target 22, the sterilization effect is expected to be enhanced. Specifically, an acidic volatile substance is gasified to generate acid gas, and the acid gas and a gas containing the peroxynitric acid mist and the peroxynitric acid gas are supplied into the sterilization container 21 so as to be applied to the sterilization target 22. Note that the acid gas may be supplied into the second container 12 or into the second pipe 16 followed by the sterilization container 21. Examples of the acidic volatile substance include nitric acid, hydrochloric acid, carbon dioxide, hydrofluoric acid, and chloric acid.

### <Variation 1>

In the sterilization treatment section 20 illustrated in Fig. 1, the sterilization container 21 may be a container that has no air outlet 24. For example, before sterilization, air in the sterilization container 21 is removed by a vacuum pump (evacuation), an internal pressure is lowered, and an interior of the sterilization container 21 is made in a negative pressure state so as to have a pressure lower than an external pressure (atmospheric pressure). Further, the interior of the sterilization container 21 may be evacuated by a vacuum pump not only before sterilization but also during a period in which the peroxynitric acid gas is applied to the sterilization target 22 (i.e., during sterilization). This allows the peroxynitric acid gas generated in the peroxynitric acid gas generation section 10, 50 to be easily filled in the sterilization container 21, and also allows all over the sterilization target 22 to be sterilized. Further, in a case where the sterilization target 22 is in the sterilization bag 23, the peroxynitric acid gas is easily filled in the sterilization bag 23, and all over the sterilization target 22 is sterilized. Furthermore, evacuation allows the sterilization container 21 to have a larger capacity and allows the sterilization target 22 to have a larger size. Moreover, repeated evacuation during sterilization enables further sterilization of all over the sterilization target 22. In addition, by the peroxynitric acid solution being exposed to vacuum, it is expected that no salt-containing impurity is produced but only the peroxynitric acid gas is efficiently produced. According to the above-listed literature of Graham et al., there is a characteristic such that the half-time of the peroxynitric acid gas itself is extended when a low pressure is reached. Thus, repeated evacuation makes it possible to prevent or reduce a decrease in concentration of the peroxynitric acid gas, so that long-term sterilization action can be maintained.

### <Variation 2>

In Figs. 1 and 9, the peroxynitric acid gas generation section 10, 50 and the sterilization treatment section 20 are separately formed, but may alternatively be integrally formed. For example, the peroxynitric acid gas generation section 10 is provided on a lower side of the interior of the sterilization container 21, and the sterilization target 22 is provided on an upper side of the interior of the sterilization container 21. Further, the peroxynitric acid gas may be generated by placing the sterilization target 22 and the peroxynitric acid solution in the sterilization container 21, and causing the interior of the sterilization container 21 to be in a negative pressure state so as to allow the peroxynitric acid solution to be easily vaporized. As in the case of Variation 1, the interior of sterilization container 21 may be repeatedly evacuated during sterilization. In that case, it is expected that no salt-containing impurity is produced but only the peroxynitric acid gas is efficiently produced. Furthermore, the peroxynitric acid gas may be generated by bubbling the peroxynitric acid solution that has been placed in the sterilization container 21.

### <Variation 3>

In Figs. 1 and 9, the peroxynitric acid gas generation section 10, 50 and the sterilization treatment section 20 are connected by, for example, the second pipe 16, but need not be connected. For example, the peroxynitric acid gas generated in the peroxynitric acid gas generation section 10, 50 is adsorbed to a porous material such as silica gel and activated carbon. Subsequently, the porous material is placed in the sterilization container 21 of the sterilization treatment section 20, and the peroxynitric acid gas is released from the porous material. Efficient sterilization can be carried out by, for example, adsorbing the peroxynitric acid gas to the porous material at a low temperature at which the half-time is extended, and releasing the peroxynitric acid gas in a short time at a high temperature.

### <Variation 4>

The sterilization treatment section 20 may include a movement means for moving the sterilization target 22, such as a belt conveyor. It is possible to carry out efficient sterilization by moving, through a space in which the peroxynitric acid gas is drifting, the sterilization target 22 the temperature of which is raised. For example, a heating zone and a sterilization zone are sequentially placed on a movement path for the movement means, the temperature of the sterilization target 22 is raised in the heating zone, and the peroxynitric acid gas is applied to the sterilization target 22 in the subsequent sterilization zone.

### <Variation 5>

A surface temperature of the sterilization target 22 may be increased. For example, the surface temperature of the sterilization target 22 is increased by continuously irradiating the sterilization target 22 with infrared light of an infrared lamp. An increase in surface temperature brings about an effect of increasing the sterilization rate (i.e., chemical reaction rate). This makes it possible to efficiently sterilize the sterilization target 22.

### <Variation 6>

In order to enhance microbicidal activity, it is possible to acidify the surface of the sterilization target 22. For example, carbon dioxide is filled in the sterilization container 21 as pretreatment, and the surface of the sterilization target 22 is acidified. Further, a carbon dioxide concentration of air to be sent to the second container 12 may be increased in the peroxynitric acid gas generation section 10. Furthermore, the carbon dioxide concentration of air to be sent to the second nozzle 52 may be increased in the peroxynitric acid gas generation section 50.

As described above with reference to Figs. 1 to 10, the peroxynitric acid that is gasified greatly expands a range of application of sterilization as compared with the peroxynitric acid solution that is as it is. For example, it is possible to sterilize the sterilization target 22 that is in the sterilization bag 23 which prevents a liquid from passing therethrough. Further, it is possible to sterilize a precision machine that cannot be sterilized in an autoclave, such as an endoscope, a heat-sensitive plastic product, and even a living body. Furthermore, since the sterilization target 22 is not wet and there are few residues, it is possible to easily sterilize, for example, a food container. Moreover, since uniform sterilization of all over a space is achieved, it is possible to easily sterilize, for example, a cell culture isolator, an operating room, a food manufacturing plant, and a plant factory. In addition, since the peroxynitric acid gas has a short half-time, detoxification treatment after sterilization is unnecessary. This makes it possible to reduce cost and time.

Use of the peroxynitric acid mist that generates the peroxynitric acid gas makes it possible to further reduce cost of a chemical solution to be used, as compared with a case where the peroxynitric acid solution is used. For example, a 100 L chemical solution is necessary in order to sterilize a 100 L container with only the peroxynitric acid solution. Further, in a case where the peroxynitric acid solution is directly sprayed, in shower form, on a sterilization target that is kept in the 100 L container, it is assumed that little peroxynitric acid gas is generated. Thus, a certain amount (e.g., several L) of chemical solution is necessary in order to completely wet the sterilization target. In contrast, use of the peroxynitric acid mist that generates the peroxynitric acid gas makes it possible to significantly reduce the amount of the chemical solution used. This consequently makes it possible to reduce the cost of the chemical solution. As a specific example, for the 90 L large sterilization container 21 illustrated in Fig. 2 to 5, the chemical solution is used in an amount of approximately 15 mL per minute in the peroxynitric acid gas generation section 10. Thus, a quite small amount of the chemical solution used is sufficient for the capacity of the container. As described earlier, the peroxynitric acid solution that has a high molar concentration is difficult to atomize, Thus, depending on the molar concentration, a much smaller amount of the chemical solution may actually act on sterilization. Further, since the peroxynitric acid mist adheres to inner surfaces of, for example, the second container 12 and the second pipe 16, a much smaller amount of the chemical solution may actually act on sterilization.

The present invention is not limited to the embodiments above, but can be put into practice in various aspects within the spirit of the present invention.

### Industrial Applicability

The present invention can be applied to sterilization of, for example, medical instruments, cell culture isolators, living organisms, and food containers, and is industrially applicable.

### Reference Signs List

1 Sterilization device
10, 50 Peroxynitric acid gas generation section
11 First container
12 Second container
13 Ultrasonic transducer
14, 56 Control device
15 First pipe
16 Second pipe
20 Sterilization treatment section
21 Sterilization container
22 Sterilization target
23 Sterilization bag
24 Air outlet
30, 55 Compressor
51 First nozzle
52 Second nozzle
53 Pump
54 Container

## Claims

1. A sterilization method wherein peroxynitric acid gas is applied to a sterilization target to sterilize the sterilization target.

2. The sterilization method as set forth in claim 1, wherein the peroxynitric acid gas is vaporized gas generated from a peroxynitric acid mist obtained by atomizing a peroxynitric acid-containing liquid.

3. The sterilization method as set forth in claim 2, wherein the peroxynitric acid mist is generated by atomizing the peroxynitric acid-containing liquid by ultrasonic atomization.

4. The sterilization method as set forth in claim 3, wherein ultrasonic waves at kilohertz frequencies are used to deaerate the peroxynitric acid-containing liquid, and ultrasonic waves at megahertz frequencies are used to atomize the deaerated peroxynitric acid-containing liquid.

5. The sterilization method as set forth in claim 3, wherein the peroxynitric acid-containing liquid is vacuum-deaerated, and the vacuum-deaerated peroxynitric acid-containing liquid is atomized by the ultrasonic atomization.

6. The sterilization method as set forth in claim 2, wherein the peroxynitric acid mist is generated by atomizing the peroxynitric acid-containing liquid by spraying the peroxynitric acid-containing liquid.

7. The sterilization method as set forth in claim 6, wherein heated air is brought into collision with the peroxynitric acid-containing liquid to generate the peroxynitric acid mist and the peroxynitric acid gas.

8. The sterilization method as set forth in claim 6, wherein the peroxynitric acid-containing liquid is sprayed in a space supplied with heated air.

9. The sterilization method as set forth in any one of claims 2 to 8, wherein the peroxynitric acid mist is removed from a gas containing the peroxynitric acid mist and the peroxynitric acid gas, and the gas from which the peroxynitric acid mist has been removed is applied to the sterilization target.

10. The sterilization method as set forth in claim 1, wherein the peroxynitric acid gas is generated by bubbling the peroxynitric acid-containing liquid.

11. The sterilization method as set forth in any one of claims 1 to 10, wherein the peroxynitric acid gas is applied to the sterilization target that is housed in a container in a negative pressure state.

12. The sterilization method as set forth in any one of claims 1 to 11, wherein an interior of the container is evacuated in a period in which the peroxynitric acid gas is applied to the sterilization target that is housed in the container.

13. The sterilization method as set forth in any one of claims 2 to 12, wherein the peroxynitric acid-containing liquid has pH of 5.85 or lower.

14. The sterilization method as set forth in any one of claims 1 to 13, wherein carbon dioxide is applied to the sterilization target.

15. The sterilization method as set forth in any one of claims 1 to 14, wherein acid gas is applied to the sterilization target.

16. A sterilization device comprising:
a peroxynitric acid gas generation section that generates peroxynitric acid gas obtained by gasifying a peroxynitric acid-containing liquid; and
a sterilization treatment section that applies the peroxynitric acid gas to a sterilization target to sterilize the sterilization target.
